# EUROPEAN PATENT APPLICATION

(11) **EP 3 698 806 A1**
(43) Date of publication of application: **26.08.2020**
(21) Application number: 19382118.8
(22) Date of filing: 19.02.2019
(51) Int. Cl.: A61K 38/48, A61P 35/00

(54) **CHEMICAL SCALPEL**

(71) Applicant: Fundacion Instituto De Investigacion Sanitaria Fundacion Jimenez Diaz, 28040 Madrid (ES); Universidad Autonoma de Madrid, 28049 Madrid (ES)
(72) Inventor: Guadalajara Labajo, Héctor, 28040 Madrid (ES); García Arranz, Mariano, 28040 Madrid (ES); García Olmo, Damián, 28049 Madrid (ES); Barambio Buendía, Javier Jesús, 28040 Madrid (ES); Cortés Guiral, Delia, 28040 Madrid (ES); Olmedillas López, Susana, 28040 Madrid (ES); Vega Clemente, Mª Luz, 28040 Madrid (ES)
(74) Representative: Hoffmann Eitle

(57) **Abstract**

The present invention relates to a liquid collagenase solution which will generate the pre-conditioning of the tissues covering peritoneal tumors by means of washing with said solution for a predetermined time and at a predetermined concentration for use as cytotoxic drug-enhancing or adjuvant treatment in the treatment of solid peritoneal tumors. Throughout the present invention, said solution will be used for administration to or for irrigating the tumor directly at a concentration and for a time such that it causes thinning of the mesothelial layer covering the intestinal tract without the involvement of intermediate or internal layers.

## Description

### Technical field of the invention

The present invention relates to the medical field, particularly to a composition comprising collagenase in solution for use as cytotoxic drug-enhancing or adjuvant treatment in the treatment of solid tumors.

### Background of the invention

The present invention is intended mainly for improving clinical results in peritoneal carcinomatosis treatment. Intraperitoneal chemotherapy is the best way to treat most of these advanced cancer stages. To obtain good results in terms of disease-free interval and survival, it is essential to achieve complete cytoreduction (extirpation of all cancerous masses) by means of complex surgical procedures. However, the surgeon might not be able to detect microscopic tumor implants which may develop after surgery. At times, microscopic tumor implants are located in positions that are very hard to access and detect. Moreover, these microscopic implants can usually be covered by layers of peritoneal mesothelial cells which may render that chemotherapy which cannot effectively reach the tumor masses ineffective.

The present invention addresses this problem by proposing a composition comprising collagenase in solution for use as cytotoxic drug-enhancing or adjuvant treatment in the treatment of solid tumors.

### Brief description of the drawings

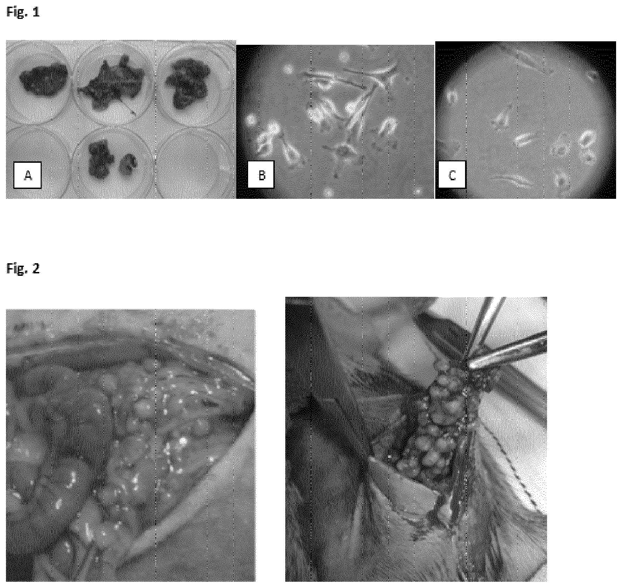

**Figure 1****.** Cells obtained from tumor tissues originating from patient biopsies cultured and treated *in vitro* with collagenase at 96 hours. A) Implant sample on dishes; B) Example of mesothelial cells released by collagenase when the tissues are digested for 30 minutes; C) Example of cells released when the tissues are digested for 24 hours with collagenase, the release of cells with tumor morphology and highly refringent mesothelial cells (a sign that the cells are detaching from the culture dish and possibly dying) is observed.
**Figure 2****.** Example of peritoneal carcinomatosis animal model (rat). Various tumor nodules are observed in the peritoneal cavity of the animals.

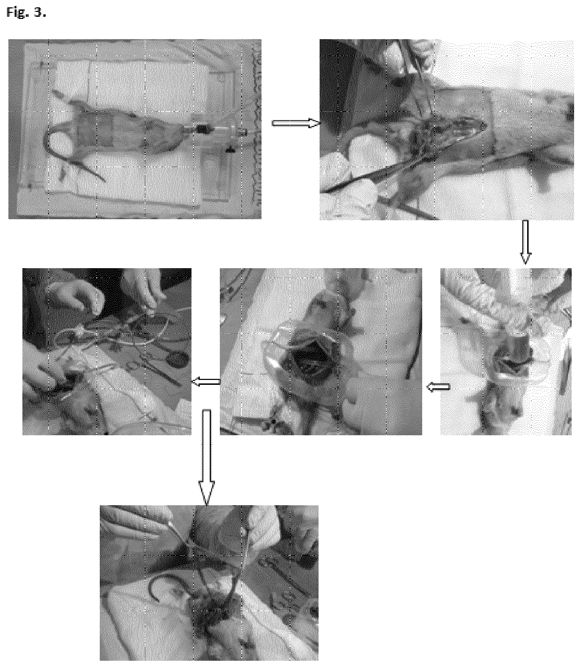

**Figure 3****.** General diagram of treatment with collagenase in a peritoneal carcinomatosis animal model. Once the animal is under anesthesia and the peritoneal cavity containing the disseminated tumor masses is exposed, washing is performed with collagenase until the cavity is full, the solution with collagenase is then recirculated at a controlled rate and temperature, finally the solution is removed, the peritoneum is washed with Ringer's lactate solution, and the peritoneum is analyzed before closing the abdomen of the animal again for follow-up.

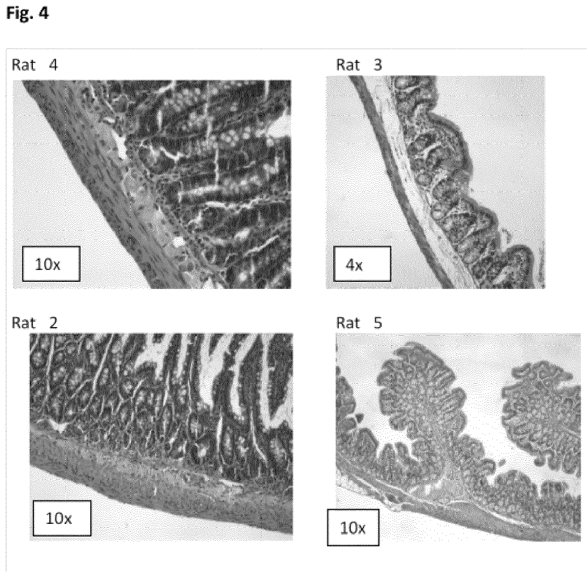

**Figure 4****.** Histological sample analysis by means of hematoxylin and eosin stain during the tests performed for fine-tuning collagenase concentration and exposure time in peritoneal washes in the peritoneal carcinomatosis animal model. As can be seen in the drawing shown, an increase in desquamating cells in intestinal sections is only observed in the case of rats 3 and 5 (35 U/mL and 87.5 U/mL, respectively); nevertheless, the inner layers of the intestine (submucosa, mucosa, and epithelium) are not affected at all, although the concentration of 87.5 U/mL does locally disrupt the submucosa, without affecting the crypts; in the case of rats from groups 2 and 4 (8.75 U/mL and Ringer's lactate solution), desquamation of the external muscle layer associated with peritoneal washing and at the low enzyme concentration is virtually inexistent, indicating that no adverse effect is caused at those concentrations and at the selected time.

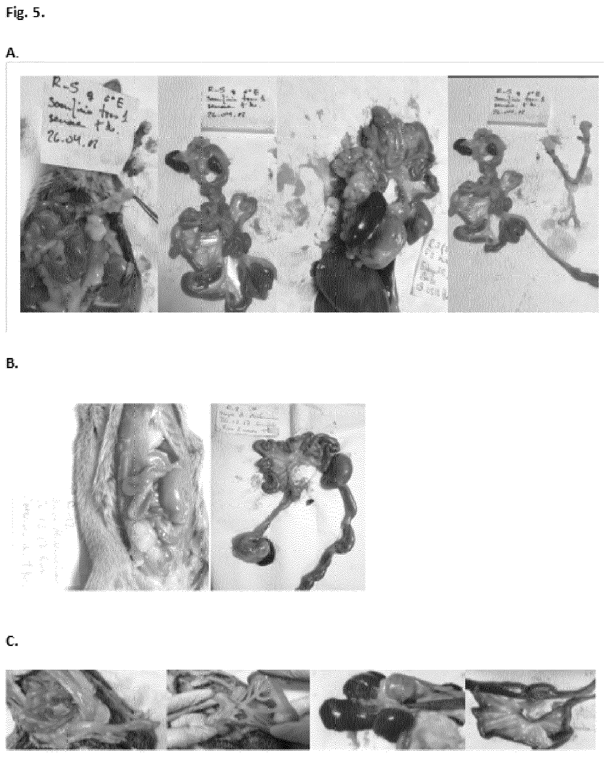

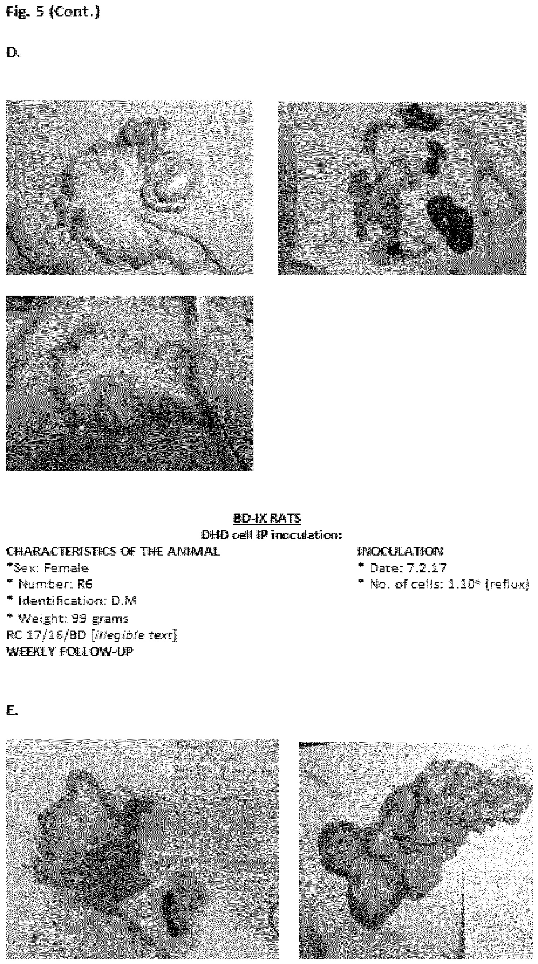

**Figure 5****.** Necropsies of the different groups of treatment while testing in a peritoneal carcinomatosis animal model:
   **5.A.** Examples of necropsies of healthy rats from the group with mitomycin 5 mg treatment after 1 week of follow-up. As the main findings, it is observed that all the abdominal organs are in a relaxed and retracted state (with kidneys that appear necrotic and liver that appears collapsed), the omentum can be ruptured upon contact, and the large intestine obstructed.
   **5.B.** Examples of necropsies of healthy rats from the group with mitomycin 0.1 mg treatment after 2 months of follow-up. As the main finding, it is observed that the intestine and an omentum are hematic.
   **5.C.** Example of necropsy of healthy rats treated with collagenase 74 U/mL and sacrificed after 2 months. The non-involvement of the abdominal organs and the omentum can be seen as the main findings.
   **5.D.** Examples of necropsies of rats with peritoneal cancer from the group with collagenase 37 U/mL + mitomycin 0.5 mg treatment after 1 week of follow-up. As the main findings, it can be observed that the organs are well preserved, and the omentum looks normal.
   **5.E.** Example of necropsy of rats with peritoneal cancer from the group with DHD treatment after 1 month of follow-up. Several tumor masses associated with the omentum and abdominal wall can be seen in the ascitic intestine.
   **5F.** Example of peritoneal cancer plus collagenase. Group F was analyzed when performing fine-tuning and the results were similar to those of group E (see Table 3).

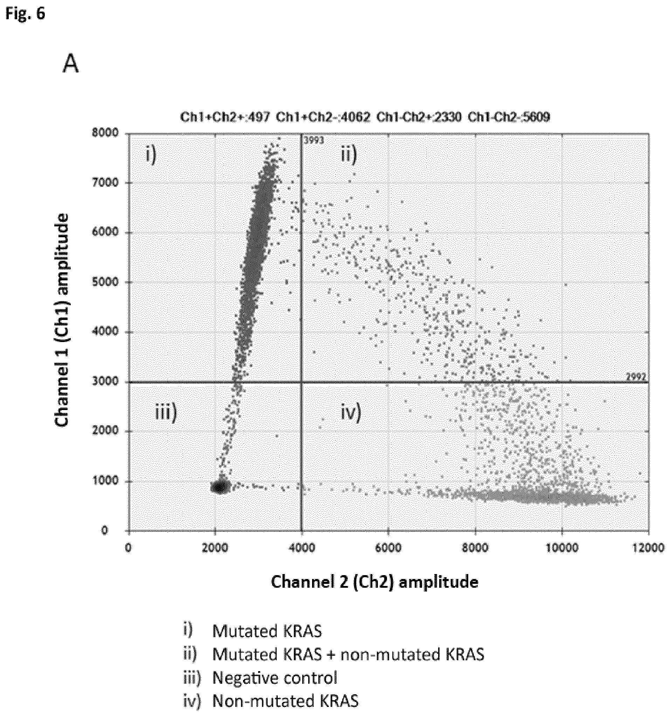

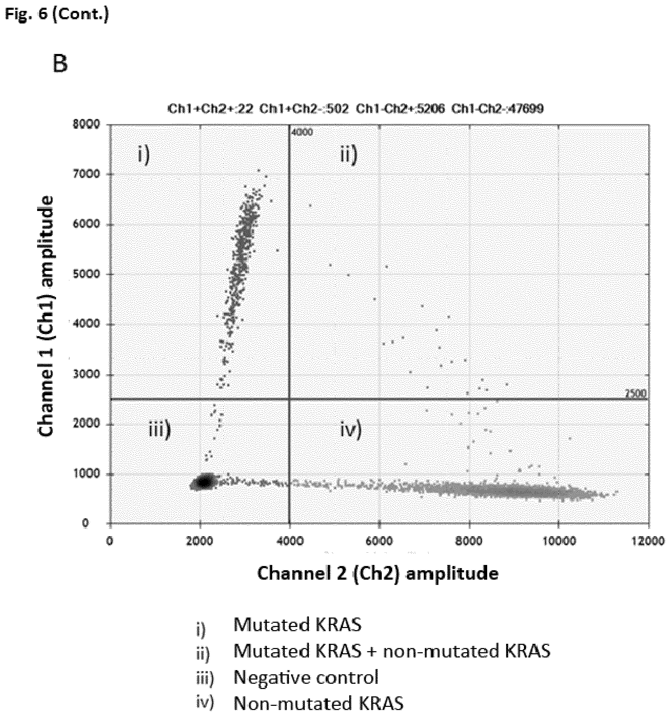

**Figure 6****.** Graphs representative of mutated and non-mutated KRAS oncogene detection by means of digital PCR. A) Example of positive control, plasma from rats with peritoneal cancer (subcutaneous DHD); B) plasma from rats with peritoneal cancer (subcutaneous DHD) treated with mitomycin C at 5 mg/animal, the only one that yields positive mutated KRAS values in plasma. Mutated KRAS (i); Mutated KRAS+ non-mutated KRAS (ii); Negative control (iii); Non-mutated KRAS (iv).

### Description of the invention

### Definitions

In the context of the present invention, "double knock-out" mice are understood to be mice modified by means of genetic engineering so that their genes are inactivated, to that end the expression or activation of the target genes is blocked

In the context of the present invention, reference is made to a liquid collagenase solution which will generate the pre-conditioning of the tissues covering peritoneal tumors by means of washing with said solution for a predetermined time and at a predetermined concentration. Throughout the present invention, said solution will be used for administration to or for irrigating the tumor directly at a concentration and for a time such that it causes thinning of the mesothelial layer covering the intestinal tract without the involvement of intermediate or internal layers. It must be noted that in the context of the present invention, said collagenase solution is defined within a specific concentration range, preferably diluted in a saline solution.

In the context of the present invention, "*DHD cells*" are understood to be the rat colon carcinoma cell line with an epithelial morphology used for studying colon metastasis in rats and for generating pseudomyxomas (peritoneal carcinomas characterized by the presence of mucinous intraperitoneal tumors).

In the context of the present invention, "syngeneic BDIX rats" are understood to be a strain of rats created in 1937 by Dr. H. Druckrey (Druckrey H., Landschutz C., and Ivankovic S. (1970) Transplacentare Erzeugnung maligner Tumoren des Nervensystems. II. Athyl-nitrosoharnstoff an 10 genetisch definierten Ratten-stammen. Z. Krebsforsch. 73, 371-386) from the cross between brother-sister rats BDI and BDVIII having the same genes and tissues, and the endogamy between the brother-sister rats has been maintained since then. This model developed by the mentioned research team allows knowing the progression times and concentration of cells to be injected for reproducing human peritoneal carcinomatosis or pseudomyxoma in rat, particularly at the anatomical pathology level.

In the context of the present invention, *"composition comprising a liquid collagenase solution which is administered to or irrigates the tumor directly at a concentration and for a time such that it causes exposure of the tumor tissue without the involvement of any organ"* is understood to mean washing the peritoneum with collagenase under controlled conditions and concentration to break up the mesothelial tissue surrounding the tumor masses and allow subsequent treatment by means of locally administered chemotherapeutics. To analyze the non-involvement of adjacent organs, anatomical-functional data demonstrating the histological non-involvement of the organs and their normal functional activity is analyzed after the proposed treatment.

In the context of the present invention, "said administration to or irrigation of the tumor directly at a concentration and for a time such that it causes thinning of the mesothelial layer covering the intestinal tract without the involvement of intermediate or internal layers," is understood to mean the intestinal tract not undergoing any anatomical-functional impairment, defining functionality as normal organ activity.

In the context of the present invention, "solid tumors" are understood to be abnormal tissue masses that generally do not contain areas with cysts or liquids. Solid tumors can be benign (non-cancerous) or malignant (cancerous). The names of the different types of solid tumors come from the type of cells forming them. Sarcomas, carcinomas, and lymphomas are examples of solid tumors.

In the context of the present invention, *"administered before chemotherapy treatment"* in reference to the collagenase solution is understood to mean washing the peritoneal region containing solid tumors or micro-tumors by means of collagenase in a controlled manner for the purpose of conditioning the tissues surrounding the tumor masses and facilitating access of the cytostatic products administered thereafter. It must be noted that, preferably and prior to administration of the cytotoxic drug or chemotherapy, the collagenase solution is inhibited.

In the context of the present invention, "cytotoxic drug-enhancing or adjuvant treatment in the treatment of solid tumors" is understood to mean treatment which allows the cytotoxic drugs to have better access to the tumor and adjacent tissues to be protected by focusing the action of the cytotoxic drugs on the tumor tissue.

In the context of the present invention, a dot is used to separate the integer part from the decimal part.

In the context of the present invention, an enzyme unit (symbol U) is defined as the catalytic activity responsible for converting one µmol of substrate per minute under optimum enzyme conditions. It is also used in combination with other units (U/mg of protein or U/mL) to indicate the specific enzymatic activity or the enzymatic activity concentration, respectively.

In the context of the present invention, a solution is a homogeneous mixture of two or more substances. The dissolved substance is called a solute and the substance in which the solute is dissolved is called a solvent.

In the context of the present invention, a suspension is a heterogeneous mixture formed by a powdered solid or small non-soluble particles (dispersed phase) which are dispersed in a liquid medium (dispersing phase).

Cytotoxic drug-enhancing or adjuvant treatment is understood to mean that pre-treatment with collagenase before treatment with the cytotoxic drug allows improving the course of the disease, enhancing the actual chemotherapy effect by exposing tumor cells to the action of intraperitoneal chemotherapy and providing a protective effect against the toxicity shown by chemotherapy treatment itself.

### Description

First, the present invention describes a new therapeutic methodology consisting of performing peritoneal washing with a collagenase enzyme solution for the purpose of treating peritoneal carcinomatosis, where said peritoneal washing must be performed before the intraperitoneal application of chemotherapy and preferably after the surgical extirpation (cytoreduction) of accessible solid tumors. In this sense, based on the experiments shown in the examples of the present patent specification, it can be concluded that performing peritoneal washing in a human subject affected by peritoneal carcinomatosis with a collagenase solution, preferably between 3,000 and 5,000 cc (cubic centimeters) of solution, and at a concentration between 37 U/mL (units per milliliter) and 148 U/mL, for a time interval between 5 and 45 minutes, and at a mean temperature between 36.5 and 39ºC, allows complete desquamation of mesothelial cells from the peritoneal surface and the microscopic tumor implants which ,may remain after the cytoreduction performed during surgery. All the tumor cells are thereby exposed the action of intraperitoneal chemotherapy. Additionally, this form of pre-treatment has the added advantage of said pre-treatment being able to act on peritoneal adhesions, making "cell shelter" spaces accessible.

This method is called "chemical peritonectomy," and the present invention proposes using same as pre-conditioning for all forms of intraperitoneal chemotherapy including HIPEC (hyperthermic intraperitoneal chemotherapy) or PIPAC (pressurized intraperitoneal aerosol chemotherapy) (HIPEC, PIPAC, etc.).

In the results obtained from the experiments performed for treating peritoneal carcinomatosis, attention is drawn to how the use of collagenase as pre-conditioning for chemotherapy treatment (see group D in Table 3 of the examples) not only enhances the cytotoxic effect, and therefore the therapeutic effect of intraperitoneal chemotherapy, but also provides a protective effect against the toxicity shown by the chemotherapy treatment itself, in this case against the high toxicity shown by mitomycin C. That is important given that, as derived from Table 3 referring to the treatment of group B2, a control group which is exclusively treated with 0.1 mg of mitomycin C, organs with thinning and bloody walls as well as many adhesions are observed. Moreover, in the necropsies of group B1, a control group treated with only 5 mg of mitomycin, many irritated and bloody adhesions, and thin organ walls and vessels are observed. Additionally, it can be seen that all the animals of group E died. In other words, high mitomycin C concentrations kill the animals, and even low mitomycin C concentrations of about 0.1 mg of mitomycin C cause thinning and bloody walls, as well as many adhesions. In stark contrast to this data, the necropsies of group D in which pre-conditioning with collagenase was performed prior to treatment with 0.5 mg or 0.1 mg of mitomycin C show the survival of all the animals and no adhesions, although carcinomatoses which are highly localized primarily in the intestine, also affecting muscle and kidneys, are observed. In other words, if the data of groups B, E, and D from Table 3 is compared, it can be observed that pre-conditioning the animals with collagenase not only allows improving the course of the disease, but also enhances the actual chemotherapy effect, the collagenase therefore acting as adjuvant treatment or treatment that enhances chemotherapy treatments such as mitomycin C, mainly for the purpose of treating peritoneal carcinomatosis, and as a protective agent against the toxicity shown by the chemotherapy treatment itself, in this case with mitomycin C.

Based on these results, a first aspect of the present invention relates to a composition comprising collagenase in solution or suspension, optionally in suspension or solution as a result of re-hydrating a composition comprising lyophilized collagenase, for use as cytotoxic drug-enhancing or adjuvant treatment in the treatment of solid tumors, where said composition comprising collagenase is administered prior to cytotoxic treatment, said solution or suspension comprising collagenase being administered to or irrigating the tumor directly. Preferably, said cytotoxic drugs are administered to or irrigate the tumor directly, and said composition comprising collagenase is administered prior to cytotoxic treatment.

In a preferred embodiment of the first aspect of the invention, the solid tumors are preferably selected from peritoneal tumors, more preferably from the list consisting of stomach adenocarcinomas or peritoneal carcinomatosis.

In another preferred embodiment of the first aspect of the invention or any of its preferred embodiments, said cytotoxic drug is selected from the list consisting of antibiotics such as mitomycin or mitomycin C, anthracyclines, bleomycins, or mithramycins, alkylating agents, antimetabolites such as 5-fluorouracil, platinum derivatives such as cisplatin or carboplatin, hormones such as androgens, antiandrogens, and estrogens, doxorubicin, or paclitaxel, interferons, interleukins, and monoclonal antibodies. Preferably, the cytotoxic drug is mitomycin, more preferably mitomycin C.

It must be noted that "being administered to or irrigating the tumor directly" is understood to mean washing the region or cavity of the organism in which the tumor is located.

In another preferred embodiment of the first aspect of the invention or any of the preferred embodiments thereof, said composition is administered to or irrigates the tumor directly at a concentration and for a time such that it causes exposure of the tumor tissue without the involvement of any organ. It must be noted that said administration to or irrigation of the tumor directly at a concentration and for a time such that it causes exposure of the tumor tissue without the involvement of any organ is preferably performed with a collagenase solution between 37 U/mL and 148 U/mL, preferably between 37 U/mL and 74 U/mL, optionally in a volume between 1,000 and 10,000 cc, preferably between 3,000 and 5,000 cc, leaving said solution to act for a period of time between 2 and 45 minutes, preferably between 7 and 12 minutes, before using a collagenase inhibitor, such as collagen, prior to administration of the cytotoxic drug.

In another preferred embodiment of the first aspect of the invention or any of the preferred embodiments thereof, said solid tumors are peritoneal tumors, and said composition is administered to or irrigates the tumor directly at a concentration and for a time such that it causes thinning of the mesothelial layer covering the intestinal tract without the involvement of intermediate or internal layers.

It must again be noted that said administration to or irrigation of the tumor directly at a concentration and for a time such that it causes thinning of the mesothelial layer covering the intestinal tract without the involvement of intermediate or internal layers is preferably performed with a collagenase solution between 37 U/mL and 148 U/mL, preferably between 37 U/mL and 74 U/mL, optionally in a volume between 1,000 and 10,000 cc, preferably between 3,000 and 5,000 cc, leaving said solution to act for a period of time between 2 and 45 minutes, preferably between 7 and 12 minutes, before using a collagenase inhibitor, such as collagen, or an inhibition based on a solution such as a Ringer's lactate solution, prior to administration of the cytotoxic drug.

A second aspect of the invention relates to a device suitable for perfusing between 1,000 and 10,000 cc, preferably between 3,000 and 5,000 cc, of collagenase solution into the peritoneal cavity of a human subject at a mean temperature between 36.5º and 39ºC, additionally characterized in that said device is suitable for recirculating the solution, preferably at a rate of 1 L/min.

A third aspect relates to a kit comprising the device according to the second aspect of the invention, where said kit further comprises an optionally lyophilized collagenase composition at a concentration such that when it irrigates the tumor directly in the peritoneal cavity of a human subject for a period of time between 2 and 45 minutes in a volume between 1,000 and 10,000 cc, preferably between 3,000 and 5,000 cc, it causes exposure of the tumor tissue without the involvement of any organ. Preferably, said rehydrated collagenase composition or collagenase composition in solution or in suspension is between 37 U/mL and 148 U/mL, preferably between 37 U/mL and 74 U/mL, in a volume between 1,000 and 10,000 cc, preferably between 3,000 and 5,000 cc.

In a fourth aspect of the invention, the device of the second or third aspect of the invention is characterized in that said device is pre-loaded with a collagenase solution at a concentration such that when it irrigates the tumor directly in the peritoneal cavity of a human subject for a period of time between 2 and 45 minutes in a volume between 1,000 and 10,000 cc, preferably between 3000 and 5000 cc, it causes exposure of the tumor tissue without the involvement of any organ. Preferably, said collagenase solution comprises between 3,000 and 5,000 cc of collagenase solution at a concentration between 37 U/mL and 148 U/mL, preferably between 37 U/mL and 74 U/mL.

A fifth aspect of the invention relates to the device or kit according to any of the preceding aspects, where said device is used in the treatment methodology specified in the first aspect of the invention or in any of the preferred embodiments thereof.

The present invention is illustrated in the following examples. It must be noted that said examples do not limit the present invention.

### Materials and methods

### In vitro studies

After obtaining authorization from the Committee for Research with Medicinal Products (PIC 75/2016_ FJD) of Fundación Jiménez Díaz, the tissue originating from patients with peritoneal carcinomatosis who signed the consent was collected. Tissue fragments were included in multiwell plates and treated with 35 U/mL of collagenase for 30 minutes or for a period of 24 hrs, the cultures were then washed and cells obtained from the enzyme treatment were analyzed. The idea was to check at different times if mesothelial cells were obtained alone or tumor cells were obtained as well. Figure 1.

### In vivo studies

### STUDIES ON THE ADJUSTMENT OF DOSE/VOLUME/TIME WITH COLLAGENASE AS ADJUVANT:

### Fine-tuning:

### First tests of peritoneal washing with collagenase on mouse:

Six 5-week old double knock-out female mice (LIPDKO), from which the lipocalin-2 gene (which affects neutrophil recruitment) and the ApoE gene (which makes it more probable for the mice to develop atherosclerosis) have been eliminated, were used. Three tubes with different concentrations of collagenase were prepared: 350 U/mL, 87.5 U/mL, and 35 U/mL in sterile PBS (buffer solution). Each concentration was injected to 2 mice according to their weight.

### First tests of peritoneal washing with collagenase on rat:

For the purpose of fine-tuning the volume of the cavity and the optimum treatment time so that no organ in the cavity will sustain any damage, twenty 6-week old Wistar rats weighing 250 grams were used, and they were tested at different concentrations of 35 U/mL of collagenase and 37 U/mL of collagenase for clinical use. Up until now, all the studies had been performed with collagenase for experimental use, nevertheless, the choice was made to use collagenase authorized for clinical use from now on, taking into account the potency equivalence calculations requested from the manufacturer. A study was then conducted using different times of treatment with collagenase from 2 minutes to 30 minutes (30, 20, 15, 10, 5, and 2) with n= 2 in the 4 intermediate times.

### Brief summary of the fine-tuning tests:

For fine-tuning the methodology of the present invention, different collagenase enzyme doses (350 U/mL, 175 U/mL, 87.5 U/mL, 70 U/mL, 35 U/mL, 17.5 U/mL, 8.75 U/mL, 3.5 U/mL, and 0%) were tested on LIPDKO mice by means of a 10-minute peritoneal washing. Cells obtained from washing which grew in culture dishes as well as the morphology of the peritoneal tissues after washing at different concentrations were analyzed. It was determined that the most suitable dose was 35 U/mL of collagenase.

Once the enzyme dose was selected, the actuation time was determined in the same manner and on the same model by means of tests from 40, 30, 20, 10, and 5 minutes. Throughout this fine-tuning method, the washing liquid was collected and cultured in a complete medium to analyze the cells obtained; different organs of the animals were also collected at the same time to check for the existence at any time (concentration/time) of any organ damage and to check the condition of the peritoneal tissues .

Finally, a comparative study was conducted between peritoneal washing with continuous external access (open washing) or using a closed system with a perfusion pump and temperature control similar to that used in an operating room. Different tests were performed on washing liquid perfusion rate and collection systems with this closed system.

### Peritoneal carcinogenesis model

The peritoneal carcinomatosis model was fine-tuned in the year 2001 by Dr. Garcia Olmo *et al.* by means of the intraperitoneal injection of DHD cells into syngeneic BDIX rats. (*The site of injection of tumor cells in rats does not influence the subsequent distribution of metastases.* García-Olmo DC, Garcia-Rivas M, García-Olmo D, Ontañón J. Oncol Rep. 2003;10(4):903-7*.; Orthotopic implantation of colon carcinoma cells provides an experimental model in the rat that replicates the regional spreading pattern of human colorectal cancer.* García-Olmo D, Garcia-Rivas M, García-Olmo DC, Atiénzar M Cancer Lett. 1998; 23;132(1-2):127-33). As can be observed in Figure 2 herein, this model reliably reproduces human pseudomyxoma (a type of peritoneal carcinomatosis).

### Collagenase safety/efficacy tests on rats with carcinomatosis

### Development of the "in vivo" model. Phase I on rats with peritoneal carcinomatosis (fine-tuning)

For the development of the "in vivo" model, carcinomatosis was caused in 6 BDIX rats, treatment was proposed after 4 weeks. The treatment consisted of using different concentrations (370 U/mL, 2x 148 U/mL, 74 U/mL, 37 U/mL, 0) of collagenase by means of peritoneal washing and it was performed in an open system by means of using a perfusion pump on 3 animals and without using any perfusion pump on 3 other animals (Figure 3). The collagenase dose selection was determined by prior studies of the group (first tests in mouse and in rat during fine-tuning). Of the 6 inoculated animals, 4 exhibited very extensive carcinomatosis, 2 exhibited mild carcinomatosis, and 1 of them only exhibited a tumor in the subcutaneous injection region. The method for peritoneal washing consisted of, after giving the animals inhalation anesthesia, exposing the abdominal cavity with a longitudinal incision and filling the peritoneal cavity with collagenase diluted to the different concentrations mentioned above. Continuous washing of the peritoneum (only with Ringer's lactate) was then performed; the animals were chosen randomly in each case and treatment lasted for 10 minutes in all the animals.

Washed tissue samples as well as organs were collected for histological studies in all cases (Figure 4).

### Tests on the safety of collagenase + chemotherapeutic agent on rats with carcinomatosis

### Adjusting the dose of collagenase as a co-adjuvant for the chemotherapeutic agent

Before performing the experiments described in the examples of the present invention to which reference is made, the safety of treatment with collagenase at defined times and doses was tested in the first phase of the work in order to carry out the present invention.

Subsequently, and having the defined dose, concentration, and times of the collagenase solution, the safety of the treatment as a co-adjuvant for or enhancer of chemotherapy treatment with mitomycin C, mainly for the purpose of treating peritoneal carcinomatosis, was tested. Different cytotoxic agents are used today for performing peritoneal washes during intraoperative chemotherapy treatments; nevertheless, evidence is proving that mitomycin C is the most commonly used cytotoxic drug and demonstrates the highest efficacy.

In this sense, after the inoculation of DHD cells in all the animals, and therefore the induction of solid tumors in the peritoneum (peritoneal carcinomatosis), the optimum dose of collagenase in terms of safety for use thereof as a co-adjuvant for or enhancer of chemotherapy treatment with mitomycin C in various work groups was determined:
- Control group: washed only with Ringer's lactate. The mesoderm readily separates and washing is non-hematic. Compact omentum.
- Group 1: 370 U/mL of collagenase. Hematic washing liquid, destruction of the omentum and mesoderm. Tumor fragments in the washing liquid.
- Group 2: 148 U/mL of collagenase. Slightly hematic washing liquid, readily separable omentum and mesoderm. Solid tumor fragments are observed in the washing liquid.
- Group 3: 74 U/mL of collagenase. Non-hematic washing liquid, readily separable omentum and mesoderm. Few implants are observed in the washing liquid but the accessibility of the tumor implants in the mesoderm and omentum is absolute.
- Group 4: 37 U/mL of collagenase. Non-hematic washing liquid, readily separable omentum and mesoderm. No tumor implants observed in the washing liquid, but they are seen to be completely exposed in both the mesoderm and omentum.

The surgical procedures during this phase consisted of a first method of peritoneal cancer induction in which DHD cells were injected into the peritoneum and 6-8 weeks were allowed to elapse until the checking for the existence of tumor masses in the peritoneum by means of abdominal palpation, and a second method of treatment in which, after giving the animals inhalation anesthesia, the abdominal cavity was exposed by means of a longitudinal incision of the abdomen, the peritoneal cavity filled with the collagenase solution at the different concentrations to be tested at 37ºC (depending on the group to which each animal corresponds), and the peritoneum continuously washed by hand with gentle continuous shaking for 10 minutes; the animals were chosen randomly in each case.

### Analysis of the survival of rats with carcinomatosis with respect to different treatments

During this phase, a series of survival experiments were established with various work groups and subgroups to learn about the co-adjuvant or enhancing role of collagenase in the treatment of peritoneal tumors with chemotherapeutic agents such as mitomycin C:

**Table 1**

| **Group nomenclature** | **Subgroup** | **Experimental observations** |
|---|---|---|
| **Group C:** Mice treated with collagenase | **A** | The peritoneum was washed with 37 U/mL of collagenase for 10 minutes |
| **Group Q:** Mice treated with mitomycin C | **B1** | Peritoneal washing with 5 mg of mitomycin C/rat for 10 minutes |
| | **B2** | Peritoneal washing with 0.5 mg of mitomycin C/rat for 10 minutes |
| **Group T:** Tumor control mice | **C** | Generation of tumors (pseudomyxoma) without treatment |
| **Group TCQ:** Mice with tumor treated with collagenase and then mitomycin C | **D1** | Generation of tumors and peritoneal washing after 3 weeks by means of 37 U/mL of collagenase for 10 minutes and then treatment by washing with 0.5 mg of mitomycin C/rat for 10 minutes |
| | **D2** | Generation of tumors and peritoneal washing after 3 weeks by means of 37 U/mL of collagenase for 10 minutes and then treatment by washing with 0.1 mg of mitomycin C/rat for 10 minutes |
| **Group TQ:** Mice with tumor treated with mitomycin C | **E** | Generation of tumors and peritoneal washing after 3 weeks by means of washing with 5 mg of mitomycin C/rat for 10 minutes |
| **Group TC:** Mice with tumor treated with collagenase | **F** | Generation of tumors and peritoneal washing after 3 weeks by means of 37 U/mL of collagenase for 10 minutes |

The surgical procedure during this phase consisted of, after giving the animals inhalation anesthesia, exposing the abdominal cavity with a longitudinal incision, filling the peritoneal cavity with 37 U/mL of collagenase solution diluted with Ringer's lactate or Ringer's lactate solution or mitomycin C solution at 37ºC (depending on the group to which each animal corresponds), and continuously washing the peritoneum by hand with gentle continuous shaking; the animals were chosen randomly in each case and treatment lasted for 10 minutes in all the animals.

The procedure was performed 2 times in the TCQ group, the first time with collagenase and after 2 minutes peritoneal washing with mitomycin C at the different concentrations to be tested was repeated. Washed tissue samples as well as organs were collected for histological studies in all cases. Blood was also collected from all the animals before sacrificing them, the blood was processed within the hour following its extraction to obtain plasma and to analyze, on one hand, the existence of the mutated KRAS oncogene (mutation in codon 12 of exon 1 expressed by DHD cells), and on the other hand the existence of collagenase traces or residues in blood after the peritoneal washes. A digital PCR was used for the KRAS oncogene localization study. For the collagenase trace study, the anonymized samples were sent to Lyposmol laboratories for the performance of specific anti-MMP-1 and anti-MMP-2 ELISAS.

Each group initially consisted of 6 rats (2 x 3). In the initial design, 3 animals were sacrificed after 15 days and the rest were left until the conditions of the animals required them to be sacrificed (subcutaneous tumor growth > 0.5 cm in diameter) or a maximum of 2 months. The tests performed consisted of daily animal control according to the data collection table; blood withdrawal to obtain plasma upon sacrifice, and histological study of the abdominal wall, small intestine, large intestine (ascending, transverse, and descending), liver, spleen, kidneys, and greater omentum. Nevertheless, unfortunately not all the animals injected with DHD generated carcinomatosis and not all the animals survived the proposed times, therefore it was impossible to follow the initial design in all groups.

### Results

The following results have been obtained in a significant manner to date:

### First tests of peritoneal washing with collagenase on mouse:

It has been deduced from the *"in vitro" results* that collagenase breaks the edges of the tissues, releasing mesothelial cells and limiting tumor vascularization at concentrations of 35 U/mL. A longer enzyme time leads to the release of more cells and greater tissue damage. Furthermore, it has been observed that at times exceeding 45 minutes the tissues become damaged and the viability of the extracted cells is inhibited. Figure 1.

The main conclusion reached after several *"in vitro"* tests is that the optimum concentration of collagenase is 35 U/mL and the optimum time is 30 minutes. Considerable increases in times or concentrations cause damage to a high percentage of cells *"in vitro".*

It can be observed from *the "in vivo" results during phase I (fine-tuning) on mouse* that half an hour after the administration of different doses of collagenase as illustrated in the table below, mice 5 and 6 (which had received the highest doses) were lethargic, exhibited signs of pain and a very reduced response to handling. It could also be seen with the naked eye that the abdominal wall had become detached. Additionally, one of the mice which received 35 U/mL of collagenase responded well to handling but exhibited signs of pain. The mice with the highest doses of collagenase died within one hour. The rest of the animals were left for 24 hours with analgesia.

**Table 2**

| **ID** | ♂/♀ | **Weight g** | **PCI*** | **Collagenase concentration** | **Involvement** | **Macroscopic observations** |
|---|---|---|---|---|---|---|
| 5 | ♀ | 165 | 10 | 87.5 U/ml | Omentum. Large implant in terminal ileum. Implant in wall (right flank). Implant in inoculation area. Intestinal mesoderm. Gastrohepatic ligament. | All hematic. Omentum, mesoderm, and abdominal wall destroyed. |
| 1 | ♂ | 220 | 13 | 35 U/ml | Omentum. Intestinal mesoderm (small intestine). Wall. Inoculation area. Gastrohepatic ligament. | All rather hematic. Implants very readily detach. Mesoderm very readily breaks. |
| 3 | ♂ | 212 | 2 | 35 U/ml | Small implant in inoculation area. No carcinomatosis. | Mesoderm readily separates. Slightly hematic liquid. |
| 2 | ♂ | 233 | 10 | 8.75 U/ml | Omentum. Mesoderm. Gastrohepatic ligament. Inoculation area. | Pump is not used. Implants do not detach. Collagenase inhibited with HEMOSNOW (porcine collagen and polysaccharides) |
| 4 | ♀ | 147 | 7 | Ringer's lactate | Adenopathies and minimum implants observed in mesoderm. Omentum. Implant in inoculation area. | Mesoderm separates readily. Slightly hematic liquid. |
| 6 | ♀ | 154 | 2 | 17.5 U/ml | Small implant in inoculation area. No carcinomatosis. | Collagenase inhibited with HEMOSNOW (porcine collagen and polysaccharides). Kept alive. |

| | | | | | | |
|---|---|---|---|---|---|---|
| *PCI: Carcinomatosis index, where >10 = carcinomatosis with significant peritoneal involvement | | | | | | |

The results of the necropsy of the fine-tuning phase are summarized below:
- Mice with a dose of 87.5 U/mL of collagenase: hematic, practically transparent abdominal wall that completely comes off the skin and ruptures by itself when touched. Peritoneal washing is performed with PSS (physiological saline solution, 0.9% sodium chloride), and the washed product is seeded on a Petri dish (P100) with DMEM+10% FBS+ 1% P/S. The dish is observed under microscope after 6 hours and cells are seen adhered to the dish, and a large amount of erythrocytes are also seen. All the abdominal organs are removed. It can be seen that the organs very readily separate from one another upon removal, as if all the structures joining them together had weakened or did not exist.
- Mice with a dose of 35 U/mL of collagenase: very thin hematic abdominal wall that ruptures by itself. Very easy organ removal.
- Mice with doses of 17.5 U/mL and 8.75 U/mL: One of the mice shows a somewhat hematic abdominal wall that has experienced the least thinning. The entire intestinal mass is secured and it takes more effort to resect it. The other mouse has an abdominal wall with a normal appearance and color. The intestinal mass is well secured. All the removed organs (peritoneum, bladder, uterus, large intestine, small intestine, stomach, spleen, liver, kidneys, and large vessels) are kept in formaldehyde for subsequent anatomical pathology study.

### First tests of peritoneal washing with collagenase on rat:

It is deduced from the initial results of the *"in vivo"* fine-tuning test on rats that the concentration of 35 U/mL of collagenase is ideal for the proposed objective and the best treatment time is 10-15 minutes; after that time elimination of the mesothelial cells covering the intestine and omentum is observed but without the involvement of any organ, including the intestinal tract. Histological studies at said concentration and time demonstrated thinning of the mesothelial layer covering the intestinal tract without the involvement of intermediate or internal layers; **which is understood to be equivalent to the exposure of the tumor tissue without the involvement of any organ.** The rest of the organs which interacted with the peritoneal cavity were not affected, thinning of the abdominal cavity wall was only observed in one study, but subsequent viability without impairment of the animal has been verified (Figure 4).

Anatomical pathology report of the samples:
- *Rat 1. Thinning is observed in the outer muscle wall and some localized minimum damage is observed in certain areas. The adipocytes surrounding the intestine are indeed destroyed.*
- *Rat 2. The intestinal wall is somewhat thinner but no damage is observed.*
- *Rat 4. Appears normal.*
- *Rat 5. The rat that sustains the most damage. There are several focal points of damage and erosion in the outer muscle layer.*

As can be observed in Figure 2, an increase in desquamating cells in intestinal sections is only observed in the case of rats 3 and 5 (70 U/mL and 87.5 U/mL respectively); nevertheless the inner layers of the intestine (submucosa, mucosa, and epithelium) are not affected at all, although the concentration of 87.5 U/mL does indeed locally disrupt the submucosa, without affecting the crypts; in the case of rats from groups 2 and 4 (8.75 U/mL and Ringer's), desquamation of the external muscle layer is virtually inexistent, indicating that no effect is caused at those concentrations and at the selected time.

The second assessable result of this fine-tuning method on rats is that, according to the manufacturer's indications, the equivalence between collagenase for experimental use and collagenase for clinical use has been calculated and verified, in view of the identical results obtained in the anatomical pathology studies and in the macroscopic study of the animals and their peritoneal organs.

### Model for generating peritoneal carcinomatosis:

In the study for fine-tuning the peritoneal carcinomatosis model, the generation of peritoneal carcinomatosis after injecting DHD cells on syngeneic BDIX rats was 80% successful in all cases.

### Analysis of the survival of rats with carcinomatosis with respect to the different treatments

Once the concentration and the carcinomatosis model were generated, the safety study was conducted by associating treatment with collagenase with treatment with mitomycin C according to the groups listed above in Table 1. It is important to point out that although the equivalence in the literature recommends a dose of mitomycin C of 5 mg/animal, the initial tests performed by the present authors showed a large number of animals that reached the endpoint criteria and had to be sacrificed, despite a suitable analgesic protocol. For that reason, the dose was lowered to 0.5 mg/animal and 0.1 mg/animal in group D. In this manner and with the suitable analgesic protocol, the animals were capable of surviving up to 2 weeks in some cases.

Macroscopic and survival results associated with the different treatments as illustrated in Table 3.

**Table 3**

| **Group** | **Treatment** | **Sacrifice/No.** | **Observations** |
|---|---|---|---|
| A | Collagenase 37 U/ml | 1 week/2 | No adhesions, normal tissues |
| A | Collagenase 37 U/ml | 2 months/2 | No adhesions, normal tissues |
| A | Collagenase 37 U/ml | 24 h/1 | Evisceration |
| B-1 | Mito 5 mg | 5 days/1 | Endpoint criterion. Peritonitis and intestinal puncture |
| B-1 | Mito 5 mg | 1 week/2 | Significant weight loss. Viscera affected and many adhesions |
| B-1 | Mito 5 mg | 2 weeks/1 | Weight loss, liquid in lungs, many adhesions, and involvement of abdominal organs |
| B-2 | Mito 0.1 mg | 1 week/1 | Organs with thinning and bloody walls. Many adhesions |
| B-2 | Mito 0.1 mg | 2 months/2 | Greenish liquid in abdomen, peritonitis. Intestinal rupture. Impaired organs and high degree of adhesions. |
| B-2 | Mito 0.1 mg | 24 h/2 | Evisceration |
| C | DHD | 1 month/3 | Localized carcinomatoses. Mild tissue adhesions |
| C | DHD | 2 months/2 | Carcinomatoses affecting multiple peritoneal organs. Nodules in lung |
| D-1 | DHD + Collagenase 37 U/ml + Mito 0.5 mg | 1 week/1 | Localized carcinomatoses in intestine. No adhesions |
| D-1 | DHD + Collagenase 37 U/ml + Mito 0.5 mg | 2 weeks/1 | Localized carcinomatoses in abdomen. No adhesions |
| D-2 | DHD + Collagenase 37 U/ml + Mito 0.1 mg | 1 week/1 | Larger number of nodules localized in intestine. No adhesions |
| D-2 | DHD + Collagenase 37 U/ml + Mito 0.1 mg | 2 weeks/1 | Nodules in abdomen, no involvement of organs. No adhesions |
| E | DHD + Mito 5 mg | 72 h/3 | Endpoint criterion |
| E | DHD + Mito 5 mg | 1 week/1 | Endpoint criterion |
| E | DHD + Mito 5 mg | 2 weeks/2 | Endpoint criterion |
| F | DHD + Collagenase 37 U/ml | 1 month/3 | Localized carcinomatoses in intestine. No adhesions |
| F | DHD + Collagenase 37 U/ml | 2 months/3 | Localized carcinomatoses in abdomen with nodules in liver. No adhesions |

The following can be considered the summary of the results listed in Table 3:
- Group A (collagenase): 4 out of 5 animals survive, 1 died after surgery due to evisceration.
- Group B (mitomycin at different doses). The animals fail to survive more than 2 weeks at a high dose (5 mg/animal) despite the high amount of continuous analgesia (Tramadol SC/12h for 2 days; Buprex + Midazolan SC/12h for 1 week); the animals are always lethargic and exhibit very little activity. At the low dose (0.1 mg/animal), 1 animal is sacrificed at week 1 for comparison with those with high doses but the rest survive until the end of the test. The need for a large amount of analgesia during the first week in this low dose group must be pointed out.
- Group C (DHD): Normal animal progression according to the data of the literature, with the animals being sacrificed at their appropriate times.
- Group D (DHD + collagenase + mitomycin): 2 concentrations of mitomycin (0.5 and 0.1 mg/animal) are used and the first animals are sacrificed at their appropriate times after 1 week and the rest after 2 weeks. These sacrifice times are not chosen due to the deteriorated condition of the animals, but rather due to the need to compare their intestinal structures with animals from group B.
- Group E (DHD + mitomycin 5 mg/animal). This group was the first group of animals generated and all the animals had to be sacrificed due to endpoint criterion despite the large amount of analgesia used throughout the entire follow-up period. Half of the animals were found dead the first night after surgery, possibly due to the effect of intraperitoneal mitomycin at a dose of 5 mg.
- Group F (DHD + Collagenase): The different animals are sacrificed after 1 and 2 months (no signs of pain are observed according to the animal monitoring protocol).

Based on this data, attention is drawn to how the use of collagenase as pre-conditioning for chemotherapy treatment (group D) allows significantly reducing the concentration of the chemotherapeutic agent used, in this case mitomycin C, to obtain similar anatomical pathology results. This is very important given that as can be seen in Table 3 referring to the treatment of group B2 with 0.1 mg of mitomycin, organs with thinning and bloody walls as well as many adhesions are observed. Moreover, in the necropsies of group B1 treated with 5 mg of mitomycin, many irritated and bloody adhesions, and thin organ walls and vessels are observed. Additionally, it can be seen that all the animals of group E died. In other words, high mitomycin concentrations kill the animals and concentrations of 0.1 mg of mitomycin C cause thinning and bloody walls, as well as many adhesions. In stark contrast to this data, the necropsies of group D in which pre-conditioning with collagenase was performed prior to treatment with 0.5 mg of mitomycin show the survival of all the animals and no adhesion, although carcinomatoses which are highly localized primarily in the intestine, also affecting muscle and kidneys, are observed. In other words, if the data of B and D of Table 3 is compared, it can be observed that pre-conditioning the animals with collagenase not only allows improving the course of the disease, but also enhances the actual chemotherapy effect, the collagenase therefore acting as adjuvant treatment for chemotherapy treatments such as mitomycin C, mainly for the purpose of treating peritoneal carcinomatoses, and acting as a protective agent against the toxicity shown by the chemotherapy treatment itself, in this case with mitomycin C.

Moreover, the plasma samples obtained from the animals of the different groups were analyzed.

The plasmas that are collected and their condition are described in Table 4.

**Table 4**

| **Group** | **Treatment** | **Sacrifice/animal** | **Plasma(ml)** | **Observations** |
|---|---|---|---|---|
| A | Collagenase 37U/mL | After 1 week: R_5 | 2 | OK |
| A | Collagenase 37U/mL | After 2 months tmt: R_1 | 2 | Mild hemolysis |
| A | Collagenase 37U/mL | After 2 months tmt: R_6 | 2 | Mild hemolysis |
| B-1 | Mito 5 mg/animal | After 1 week tmt: R_11 | 1.5 | OK |
| B-1 | Mito 5 mg/animal | After 2 months tmt: R_8 | 2 | Mild hemolysis |
| B-1 | Mito 5 mg/animal | After 2 months tmt: R_12 | 2 | Mild hemolysis |
| C | DHD | After 1 month of inoculation: R_1 | 2 | OK |
| C | DHD | After 1 month of inoculation: R_4 | 3 | OK |
| C | DHD | After 1 month of inoculation: R_5 | 3 | OK |
| D-1 | DHD + Colag 37U/mL + Mito 0.5 mg | After 2 weeks tmt: R_5 | 1.6 | OK |
| D-2 | DHD + Colag 37U/mL + Mito 0.1 mg | After 1 week tmt: R_4 | 2 | OK |
| D-2 | DHD + Colag 37U/mL + Mito 0.1 mg | After 2 weeks tmt: R_5 | 2 | OK |
| E | DHD + Mito 5 mg/animal | After 1 week tmt: R_1 | 0.5 | Mild hemolysis |
| E | DHD+ Mito 5 mg/animal | After 5 days tmt: R_2 | 2.5 | Mild hemolysis |
| F | DHD + Collagenase 37U/mL | After 1 month: R_2 | 3.5 | OK |
| F | DHD + Collagenase 37U/mL | After 2 months of inoculation: R_3 | 3 | OK |

To analyze the presence of mutated KRAS in the blood of the different groups by means of digital PCR, DNA was extracted from at least 2 ml of plasma/group by means of the QIAamp Circulating Nucleic Acid kit according to the manufacturer's data and the results were those listed in Table 4. Figure 6 shows the graph obtained for group C (positive control) as well as group E which is the only group that can be considered as having positive mutated KRAS in the plasma among the different treatments.

**Table 4**

| Group | Mutated KRAS event no. | Healthy KRAS event no. |
|---|---|---|
| A | 0 | 2426 |
| B | 0 | 1253 |
| C | 13094 | 7424 |
| D-1 | 7 | 8677 |
| D-2 | 2 | 3774 |
| E | 524 | 5228 |
| F | 1 | 2198 |

The ELISA results to check if there are collagenase residues left in the blood of the animals after treatment and the follow-up period were all negative for both MMP-1 and MMP-2.

### Conclusions:

- The designed method is feasible, safe, and repeatable.
- The generated product is shown as a chemical/enzymatic scalpel.
- No organ or tissue impairment is observed histologically at the optimum concentrations and times developed in the study.
- The only animal that was left alive after treatment showed no impairments or pain.

## Claims

1. A composition comprising collagenase in solution or suspension for use as cytotoxic drug-enhancing or adjuvant treatment in the treatment of solid peritoneal tumors, wherein said composition comprising collagenase is administered prior to cytotoxic treatment, being administered to or irrigating the tumor directly.

2. The composition for use according to claim 1, wherein said cytotoxic drugs are administered to or irrigate the tumor directly, and wherein said composition comprising collagenase is administered prior to cytotoxic treatment, being administered to or irrigating the tumor directly.

3. The composition for use according to claim 2, wherein said composition is administered to or irrigates the tumor directly at a concentration and for a time such that it causes exposure of the tumor tissue without the involvement of any organ.

4. The composition for use according to claim 2 or 3, wherein the action of the collagenase is inhibited by a collagenase inhibitor, such as collagen, being administered to or irrigating the tumor directly, prior to administration of the cytotoxic drug.

5. The composition for use according to any of claims 1 to 4, wherein said cytotoxic drug is mitomycin.

6. The composition for use according to any of claims 1 to 4, wherein said cytotoxic drug is mitomycin C.

7. The composition for use according to any of claims 1 to 6, wherein said tumors are selected from the list consisting of peritoneal adenocarcinomas or carcinomas.

8. The composition for use according to claim 7, wherein said composition is administered to or irrigates the tumor directly at a concentration and for a time such that it causes thinning of the mesothelial layer covering the intestinal tract without the involvement of intermediate or internal layers.

9. The composition for use according to any of claims 1 to 8, wherein said composition comprises a collagenase solution between 37 U/mL (units per milliliter) and 148 U/mL.

10. The composition for use according to claim 9, wherein said collagenase is administered to or irrigates the tumor directly and is left to act for a period of time between 2 and 45 minutes before using the collagenase inhibitor, prior to administration of the cytotoxic drug.

11. A device suitable for perfusing between 1,000 and 10,000 cc of collagenase solution into the peritoneal cavity of a human subject at a mean temperature between 36.5 and 39ºC, additionally **characterized in that** said device is suitable for recirculating the solution, preferably at a rate of 1 L/min.

12. A kit comprising the device according to claim 11, wherein said kit further comprises an optionally lyophilized collagenase composition at a concentration such that when it directly irrigates the tumor in the peritoneal cavity of a human subject for a period of time between 2 and 45 minutes in a volume between 1000 and 10,000 cc, it causes exposure of the tumor tissue without the involvement of any organ.

13. The kit according to claim 12, wherein said rehydrated collagenase composition or collagenase composition in solution is at a concentration between 37 U/mL (units per milliliter) and 148 U/mL in a volume between 1000 and 10,000 cc.

14. The kit according to claim 12 or the device according to claim 11, **characterized in that** said device is pre-loaded with a composition comprising between 3000 and 5000 cc of collagenase solution at a concentration between 37 U/mL (units per milliliter) and 148 U/mL.

15. The device or kit according to any of claims 11 to 14, wherein said kit or device is used in the treatment methodology according to any of claims 1 to 8.
